# FASCICULE DE BREVET EUROPEEN

(11) **EP 2 231 516 B1**
(45) Date de publication et mention de la délivrance du brevet: **07.08.2019**
(21) Numéro de dépôt: 08872468.7
(22) Date de dépôt: 20.11.2008
(51) Int. Cl.: C01B 32/158, C01B 32/168, C01B 32/174, B01J 21/18, H01M 4/96, H01G 11/34, B01D 71/02, A61L 27/44, C02F 1/44

(54) **AEROGELS DE NANOTUBES DE CARBONE**
Aerogele von Kohlenstoffnanoröhrchen
AEROGELS OF CARBON NANOTUBES

(30) Priorité: 21.11.2007 FR 0708167
(43) Date de publication de la demande: 29.09.2010
(73) Titulaire: Centre National de la Recherche Scientifique (C.N.R.S.), 75794 Paris Cedex 16 (FR)
(72) Inventeur: PÉNICAUD Alain, F-33000 Bordeaux (FR)
(74) Mandataire: Novagraaf Technologies
(86) Numéro de dépôt international: PCT/FR2008/001627
(87) Numéro de publication internationale: WO 2009/101271

(56) Documents cités:
- WO-A-2005/073127
- FR-A- 2 910 458
- US-B1- 6 684 524
- BRYNING MATEUSZ B ET AL: "Carbon nanotube aerogels" ADV MATER; ADVANCED MATERIALS MAR 5 2007, vol. 19, no. 5, 5 mars 2007 (2007-03-05), pages 661-664, XP002487809 cité dans la demande
- CHEN ET AL: "A new method for the preparation of stable carbon nanotube organogels" CARBON, ELSEVIER, OXFORD, GB, vol. 44, no. 11, 1 septembre 2006 (2006-09-01), pages 2142-2146, XP005523299 ISSN: 0008-6223
- PENICAUD A ET AL: "Dissolution douce of single walled carbon nanotubes" AIP CONFERENCE PROCEEDINGS AIP USA, no. 786, 2005, pages 266-270, XP002487810 ISSN: 0094-243X
- SABBA Y ET AL: "High-concentration dispersion of single-wall carbon nanotubes" MACROMOLECULES; MACROMOLECULES JUN 29 2004, vol. 37, no. 13, 29 juin 2004 (2004-06-29) , pages 4815-4820, XP002487811
- HOUGH L A ET AL: "Viscoelasticity of single wall carbon nanotube suspensions" PHYSICAL REVIEW LETTERS APS USA, vol. 93, no. 16, 15 octobre 2004 (2004-10-15), pages 168102/1-4, XP002487812 ISSN: 0031-9007

## Description

### Domaine technique

La présente invention se rapporte à un procédé de préparation d'aérogels de nanotubes de carbone individualisés et ses applications, notamment pour la fabrication d'aérogels composites et de composés électrochimiques.

La présente invention se rapporte en particulier à des aérogels de nanotubes de carbone individualisés obtenus par ledit procédé, ainsi qu'à des utilisations de ces aérogels.

L'obtention de tels aérogels de nanotubes de carbone présente un grand intérêt pour leurs applications industrielles, en particulier en vue des propriétés uniques des nanotubes de carbone, et de la très faible densité apparente et grande surface spécifique des aérogels précités.

Dans la description ci-dessous, les références entre crochets ([]) renvoient à la liste des références présentée après les exemples.

### État de la technique

Les matériaux communément désignés par *"aérogels de carbone"* sont des matériaux macroscopiques essentiellement (et généralement exclusivement) constitués de carbone, qui présentent une structure extrêmement poreuse, entraînant une densité apparente très réduite. Pour plus de détails concernant les structures de type aérogels de carbone, on pourra notamment se reporter à l'article *"*Les aérogels et le structure alvéolaires : deux exemples de mousses de carbone" de L. Kocon et T. Piquero, dans L'actualité Chimique, N° 245-246, pp. 119-123 (mars-avril 2006) [ref 1].

Les aérogels de carbone sont généralement obtenus par des processus dits par "réplique" ("*templating"* en anglais). Dans ces procédés, schématiquement, on forme une structure tridimensionnelle poreuse de carbone ou d'un précurseur de carbone en employant une structure solide ou une organisation moléculaire de type cristal liquide à titre de "moule" de la structure recherchée. Ce "moule", dit agent texturant (*"template"* en anglais) peut prendre différentes formes selon le procédé utilisé. Dans ce cadre, il existe trois grandes familles de procédés par texturation conduisant aux aérogels de carbone : (1) l'emploi de solides micro- ou mésoporeux à titre de texturants solides ["Nouveaux concepts d'élaboration de matériaux carbonés poreux" de C. Vix-Guterl, J. Parmentier, P. Delhaés, dans L'actualité chimique, n° 245-246, pp 124-128 (mars-avril 2006) [ref 2] ; et *"*Synthesis of highly ordered carbon molecular sieves via template-mediated structural transformation" de R. Ryoo, S.-H. Soo, S. Jun, dans The Journal of Physical chemistry B, 103 (37), pp. 7743-7746 (1999) [ref 3]], (2) la texturation de carbone en milieu liquide ou gélifié ["High-thermal conductivity, mesophase pitch-derived carbon foams : effect of precursors on structure and properties" de J. Klett et col., dans Carbon, 38, pp. 153-173 (2000) [ref 4] ou "Novel high strength graphitic foams", de T. Beechem, K. Lafdi, dans Carbon, 44, pp. 1548-1549 (2002) [ref 5]] et (3) la texturation de précurseurs carbonés en milieu liquide ou gélifié ["Fabrication of nano-structure control of carbon aerogels via microemulsion templatted sol-gel polymerisation method", de D. Wu, R. Fu, M.S. Dresselhaus, G. Dresselhaus, dans Carbon, 44, pp 675-680 (2005) [ref 6] *ou "*Preparation and properties of resorcinol formaldehyde organic and carbon gels", de S.A. Al-Muthtsabeb, J.A. Ritter, dans Adv. Mater., 15(2), pp. 101-104 (2003) [ref 7]].

Cependant, aucune de ces méthodes ne concerne les aérogels de nanotubes de carbone. Or, la possibilité d'élaborer des aérogels de nanotubes de carbone présente un intérêt industriel et scientifique manifeste, en vue des propriétés mécaniques, électriques et chimiques uniques des nanotubes de carbone . En effet, ceux-ci sont couramment utilisés dans les matériaux composites (Schaffer, M. S. P., Windle, A. H., "Fabrication and Characterization of Carbon Nanotube/poly (vinyl alcohol) Composites", Adv. Mater., 11, pp 937-941 (1999) [ref 8]), les supercondensateurs (Aldissi, M.; Schmitz, B.; Lazaro, E.; Bhamidipati, M.; Dixon, B., "Conducting Polymers In Ultracapacitor Applications", 56.sup.th Annu. Tech. Conf.--Soc. Plast. Eng., (Vol. 2), pp 1197-1201 (1998) [ref 10]; An, K. H.; Kim, W. S.; Park, Y. S.; Moon, J.-M.; Bae, D. J.; Lim, S. C.; Lee, Y. S.; Lee, Y. H. "Electrochemical Properties Of High-Power Supercapacitors Using Single-Walled Carbon Nanotube Electrodes", Adv. Funct. Mater. 11, pp 387-392 (2001) [ref 11]), la catalyse (Yu, R., Chen, L., Liu, Q., Lin, J., Tan, K. -L., Ng, S. C., Chan, H. S. O., Xu, G.-Q.,Hor, T. S. A. "Platinum Deposition On Carbon Nanotubes Via Chemical Modification", Chem. Mater. 10, pp 718-722 (1998) [ref 12]; (Planeix, J. M.; Coustel, N.; Coq, B.; Brotons, V.; Kumbhar, P. S.; Dutartre, R.; Geneste, P.; Bernier, P.; Ajayan, P. M., "Application Of Carbon Nanotubes As Supports_in Heterogeneous Catalysis", J. Am. Chem. Soc. 116, pp 7935-7936 (1994) [ref 13]) et les composants ou systèmes électroniques de taille nanométrique (Tans, S. J., Verschueren, A. R. M., Dekker, C., "Room-Temperature Transistor Based On A Single Carbon Nanotube", Nature 393, pp 49-52 (1998) [ref 14]; Bachtold, A.; Hadley, P.; Nakanishi, T.; Dekker, C., "Logic Circuits With Carbon Nanotube Transistors". Science 294 pp 1317-1320 (2001) [ref 15]). Ainsi, développer un matériau aérogel à base de nanotubes de carbone présente un grand intérêt.

Deux méthodes d'élaboration de tels matériaux ont été rapportées à ce jour : (1) Yodh et al., « Carbon nanotube aerogels », Advanced Materials, 2007, 19, pp. 661-664 [ref 16] ; et (2) Backov et al., Demande de brevet français N° 06/11143 (publication N° FR 2 910 458) [ref 17]. Cependant, dans les deux cas, la méthode utilise une dispersion de nanotubes de carbone en présence de tensioactifs et une étape de sonication pour optimiser la dispersion. Cela engendre plusieurs inconvénients. D'une part, les nanotubes de carbone sont raccourcis par la sonication. Le rapport diamètre/longueur des nanotubes de carbone dans l'aérogel n'est donc pas optimal. Cela peut également se traduire par des problèmes de contacts électriques entre les nanotubes dans l'aérogel final. D'autre part, l'aérogel obtenu contient un composé tensioactif si le procédé ne prévoit pas une étape visant à le retirer. La présence de tensioactif peut nuire à la qualité de l'aérogel, et peut entraver son utilisation selon l'application envisagée. Par ailleurs, les tensioactifs sont fréquemment associés à des problèmes de mauvaise biodégradabilité. Ces méthodes présentent donc l'inconvénient qu'une étape supplémentaire d'élimination du tensioactif doive être prévue.

On pourra se référer également aux travaux de Bryning et al. ("Carbon nanotube aerogels" Adv. Mater.; Advanced Materials, 2007, vol. 9 (5), pp. 661-664) portant sur l'obtention d'un aérogel de nanotubes de carbone à partir d'une suspension aqueuse de nanotubes de carbone en présence d'un surfactant et soumise à un traitement aux ultrasons.

Par ailleurs, le document FR 2 910 458 décrit un aérogel de nanotubes de carbone obtenu par un procédé dans lequel:
- on réalise une dispersion aqueuse de nanotubes de carbone dans l'eau en présence d'un agent tensioactif dispersant,
- on réalise une mousse par foisonnement de la dispersion sous l'effet d'un gaz en présence d'un agent moussant, et
- on congèle la mousse et on élimine l'eau par sublimation.

Là encore, la dispersion aqueuse de nanotubes de carbone est réalisée à l'aide d'une sonication.

Par ailleurs, chacune des méthodes précitées utilise des nanotubes de carbone sous forme de faisceaux (i.e., ils ne sont pas individualisés). La surface spécifique de l'aérogel ainsi obtenu s'en trouve donc affectée. En tout état de cause, la surface spécifique des aérogels actuellement connus est loin d'être optimale.

Il existe donc un réel besoin d'un procédé de préparation d'aérogels de nanotubes de carbone palliant ces défauts, inconvénients et obstacles de l'art antérieur.

### Description de l'invention

La présente invention a précisément pour but de répondre à ce besoin en fournissant un procédé de préparation d'aérogels de nanotubes de carbone caractérisé en ce qu'il comprend les étapes suivantes réalisées sous atmosphère inerte:
(a) réduction de nanotubes de carbone par un métal alcalin pour conduire à un sel polyélectrolyte de nanotubes de carbone;
(b) exposition dudit sel polyélectrolyte de nanotubes de carbone à un solvant polaire aprotique pour conduire à une solution de nanotubes de carbone individualisés réduits ;
(c) congélation de ladite solution de nanotubes individualisés ; et
(d) sublimation du solvant ;
le procédé étant réalisé sans altération structurale des nanotubes de carbone par sonication et en l'absence de tensioactif.

Les étapes a) et b) du procédé sont toujours effectuées sous atmosphère inerte. Par « atmosphère inerte », on entend par la présente un gaz ou un mélange gazeux qui ne favorise pas la ré-oxydation des nanotubes de carbone réduits en nanotubes neutres. Par exemple, le procédé est conduit sous atmosphère gazeuse dépourvue d'oxygène. En particulier, le procédé peut être effectué sous atmosphère d'argon ou d'azote.

Dans un mode de réalisation, le métal alcalin peut être tout métal alcalin permettant de mettre en oeuvre la présente invention. Il peut être choisi par exemple dans le groupe comprenant le lithium, le sodium, le potassium, le rubidium et le césium. Plus particulièrement, le métal alcalin peut être le lithium, le sodium ou le potassium. Dans certains modes de réalisation particuliers, le métal alcalin est le lithium ou le sodium. Dans d'autres modes de réalisation particuliers, le métal alcalin est le potassium.

Par « réduction par un métal alcalin» on entend dans la présente une réduction dans laquelle un métal alcalin est impliqué. Ainsi, la réduction peut se faire directement en présence d'un métal alcalin, par exemple en phase vapeur. Des méthodes de réduction en présence d'un métal alcalin sont bien connues dans l'art. L'homme du métier saura identifier les conditions opératoires adéquates pour mettre en oeuvre un procédé de réduction en présence d'un métal alcalin, par exemple en phase vapeur. Notamment, l'homme du métier pourra s'inspirer des méthodes décrites dans « Synthesis of graphite intercalation compounds », A. Hérold in Chemical physics of intercalation, A.P. Legrand et S. Flandrois Eds, NATO ASI Series, series B, Vol. 172, pp. 3-45 (1987) par exemple [ref 18], qui sont directement applicables aux nanotubes de carbone.

Dans un autre mode de réalisation, la réduction se fait en présence d'un sel de métal alcalin obtenu à partir d'un métal alcalin. Par exemple, la réduction peut se faire en présence d'un sel polyaryl alcalin de formule A⁺B⁻, dans laquelle A⁺ représente un cation d'un ion alcalin, et B⁻ représente un anion d'un composé polyaromatique.De tels sels polyaryl alcalin et leur procédé de fabrication sont décrits par exemple dans (C. Stein, J. Poulenard, L. Bonnetain, J. Golé, C.R. Acad. Sci. Paris 260, 4503 (1965) [ref 19]; « Synthesis of graphite intercalation compounds », A. Hérold in Chemical physics of intercalation, A.P. Legrand et S. Flandrois Eds, NATO ASI Series, series B, Vol. 172, pp. 3-45 (1987) [ref 20]; F. Béguin et R. Setton New ternary lamellar compounds of graphite, Carbon 13, 293-)295 (1975) [ref 21] ; Pénicaud et al., « Spontaneous dissolution of a single-wall carbon nanotube salt », J. Am. Chem. Soc., 127, 8-9, (2005) [ref 34].

Selon un mode de réalisation, le composé polyaromatique est choisi dans le groupe comprenant le naphtalène, le phénanthrène, le biphényle, l'anthracène, le pérylène, la benzophénone, la fluorénone, la benzoquinone et l'anthraquinone. Dans un mode de réalisation particulier, le composé polyaromatique est le naphtalène. Dans un mode de réalisation particulier, le sel polyaryl alcalin est un sel polyaryl de potassium (c'est-à-dire, un sel de formule A⁺B⁻, dans laquelle A⁺ représente K⁺). Avantageusement, le sel polyaryl alcalin de formule A⁺B⁻, est un sel de potassium de naphtalène (Naph⁻ K⁺).

Par «nanotubes de carbone individualisés réduits» on entend dans la présente un composé comprenant au moins deux nanotubes individuels de carbone chargés négativement et neutralisés par des contre ions positifs de métal alcalin en solution dans un solvant polaire aprotique. Les nanotubes de carbone existent généralement sous forme de faisceaux de nanotubes (i.e., les nanotubes ne sont pas individuels, ils sont « agglomérés » en faisceaux). Dans la présente, l'expression «nanotubes de carbone individualisés réduits» se réfère à des faisceaux de nanotubes de carbone partiellement désagglomérés, c'est-à-dire des faisceaux de nanotubes de carbone dont la surface spécifique est accrue par rapport à celle des faisceaux de départ (avant la mise en oeuvre des étapes a) et b) du procédé de l'invention). Ainsi, l'expression «nanotubes de carbone individualisés réduits» peut représenter un mélange de nanotubes de carbones individuels et de faisceaux partiellement désagglomérés, chargés négativement et neutralisés par des contre ions positifs de métal alcalin. De préférence, les «nanotubes de carbone individualisés réduits» comportent majoritairement des nanotubes de carbones individuels chargés négativement et neutralisés par des contre ions positifs de métal alcalin. De préférence, lesdits nanotubes de carbone ne se présentent pas sous la forme de faisceaux, mais exclusivement sous la forme de nanotubes individuels.

Les faisceaux de nanotubes sont dans un premier temps réduits par un métal alcalin pour former un sel polyélectrolyte de nanotubes de carbone (étape a) du procédé de l'invention). L'individualisation (partielle, et de préférence complète) des nanotubes de carbone se fait lors de l'étape b) du procédé par exposition à un solvant polaire aprotique, qui solvate les nanotubes de carbone réduits, et les sépare ainsi les uns des autres.

Les nanotubes de carbone individualisés réduits peuvent se présenter sous la forme d'un composé binaire de formule MC_{X} où M représente un contre-ion positif d'un métal alcalin (M+), et x représente un nombre entier compris entre 6 et 200. En particulier, le métal alcalin peut être le potassium, le lithium ou le sodium.

Les nanotubes de carbone individualisés réduits peuvent se présenter sous la forme d'un composé ternaire de formule M(Solv)yCx dans laquelle M est un ion métallique alcalin (M+), Solv est une molécule de solvant aprotique, x représente un nombre entier compris entre 6 et 200, et y représente un nombre compris entre 0 et 8. La molécule de solvant peut être une molécule d'un solvant aromatique (par exemple le benzène ou le toluène) ou nucléophile (par exemple, un solvant dont la structure contient au moins un atome d'oxygène comme le THF). Par exemple, le solvant est le THF et le sel polyélectrolyte de nanotubes de carbone est un composé ternaire de structure Na(THF)yCx, Li(THF)yCx ou K(THF)yCx dans laquelle x représente un nombre entier compris entre 6 et 200, et y représente un nombre compris entre 0 et 8. Par exemple, le composé ternaire peut répondre à la formule K(THF)C₁₀, Na(THF)C₁₀, Li(THF)C₁₀ ou Li(THF)C₆. Le nombre y n'est pas forcément un nombre entier. Il peut en effet représenter une moyenne du nombre de coordination du solvant Solv sur le cation de métal alcalin. Par exemple, des composés ternaires M(Solv)yCx ont été préparés et isolés où la variable y a été mesurée comme étant égale à 0,8. Dans la présente, et par simplicité d'écriture, le nombre y est arrondi au nombre entier supérieur ou inférieur le plus proche. Par exemple, les composés ternaires de formule Na(THF)C₁₀ ou Li(THF)C₁₀ référencées ci-dessus englobent des composés dont l'analyse élémentaire a révélé qu'il s'agissait de composés de formule Na(THF)_{0,8}C₁₀ ou Li(THF)_{0,8}C₁₀. Ainsi, toute formule M(Solv)yCx référencée dans la présente doit être entendue comme représentant un composé de formule M(Solv)_{y±0,5}Cx.

Selon un mode de réalisation particulier, l'étape de réduction a) se fait en présence d'un solvant. Par exemple, le solvant peut être un solvant nucléophile. Par exemple, le solvant nucléophile peut être un solvant aprotique dont la structure contient au moins un atome d'oxygène, en particulier le THF.

Selon un mode de réalisation particulier, l'étape de réduction est choisie dans le groupe comprenant la réduction par un métal alcalin en phase vapeur suivie d'une exposition à un solvant aprotique, la réduction électrochimique et la réduction par un sel polyaryl alcalin dans un solvant aprotique. Par exemple, le solvant peut être un solvant aromatique, tel que le benzène ou le toluène. Le solvant peut être un solvant aprotique dont la structure contient au moins un atome d'oxygène comme le THF.

Selon un mode de réalisation particulier, l'étape de réduction a) comprend l'addition aux nanotubes de carbone, sous atmosphère inerte, d'un sel polyaryl alcalin de formule A⁺B⁻, dans laquelle :
A⁺ représente un cation d'un ion alcalin, et
B⁻ représente un anion d'un composé polyaromatique.

Selon un mode de réalisation, le composé polyaromatique est choisi dans le groupe comprenant le naphtalène, le phénanthrène, le biphényle, l'anthracène, le pérylène, la benzophénone, la fluorénone, la benzoquinone et l'anthraquinone.

Selon un mode de réalisation particulier, le solvant polaire aprotique utilisé dans l'étape d'exposition b) a une constante diélectrique de 25 à 200. Par exemple, le solvant polaire aprotique peut être le sulfolane, le diméthylsulfoxyde (DMSO), le diméthylformamide, la N-méthylpyrrolidone ou le N-méthylformamide. Dans un mode de réalisation particulier, le solvant polaire aprotique est le DMSO. Dans un autre mode de réalisation particulier, le solvant polaire aprotique est la N-méthylpyrrolidone.

Ainsi, le procédé de l'invention permet de préparer un aérogel de nanotubes de carbone à partir d'une solution organique polaire, par opposition aux solutions aqueuses dopées de tensioactifs utilisées dans l'art antérieur (Yodh et al. et Backov et al.).

Ceci n'est aucunement trivial, et ne découle pas d'une manière évidente de l'état de la technique.

En effet, les solvants polaires aprotiques tels que le sulfolane, le diméthylsulfoxyde (DMSO), le diméthylformamide (DMF), la N-méthylpyrrolidone (NMP) ou le N-méthylformamide ont des tensions de vapeur bien plus faible que l'eau. Par exemple, la tension de vapeur du DMSO est un ordre de grandeur plus faible que celle de l'eau à environ 0°C (à titre d'exemple, Pvap (eau) = 6 mbars à zéro degrés contre Pvap(DMSO) = 0.2 mbars à 6 degrés).

De plus, la lyophilisation étant fréquemment utilisée en biologie, à notre connaissance sur des solutions exclusivement aqueuses ou contenant un solvant organique tel que le DMSO en proportion mineure, les lyophilisateurs commerciaux sont calibrés pour l'eau. Pour cette raison, ils ne sont pas adaptés à la lyohilisation de solutions organiques, particulièrement des solvants polaires à tension de vapeur très faible comme les solvants précités.

L'homme du métier n'était donc pas encouragé à explorer la lyophilisation de solutions de nanotubes de carbone individualisés réduits dans un solvant polaire aprotique telles que celles intervenant dans le procédé de l'invention, dans la mesure où l'on n'a pas affaire à des solutions aqueuses.

Ainsi, compte tenu des difficultés techniques précitées et des caractéristiques physicochimiques des solvants polaires aprotiques (notamment leur tension de vapeur), la lyophilisation de solutions organiques polaires aprotiques avant la présente invention n'était pas évidente pour l'homme du métier.

Les inventeurs ont découvert de manière tout à fait surprenante qu'il était possible de lyophiliser une solution organique polaire aprotique, telle qu'une solution de DMSO, en particulier une solution organique polaire aprotique contenant des nanotubes de carbone individualisés.

Une difficulté est de s'affranchir des lyophilisateurs commerciaux qui, comme indiqué ci-dessus, sont calibrés pour des solutions aqueuses.

Pour les solvants plus réfractaires tels que la NMP (Tension de vapeur = 2.10⁻³ mbars à -30°C (Tcongélation= -25°C)), cette lyophilisation peut notamment être mise en oeuvre avec une pompe à vide puissante, telle qu'une pompe turbomoléculaire, voire plusieurs (au moins deux) pompes turbomoléculaires en série.

Selon un mode de réalisation particulier, l'étape d'exposition b) est effectuée à une température de -60 à 285 °C. Par exemple, l'étape d'exposition b) est effectuée à une température de 0 à 35 °C, de préférence de 20 à 25°C.

D'une manière générale, le procédé de l'invention, en particulier l'étape d'exposition b), peut être mis en oeuvre avec ou sans agitation. Lorsqu'un système d'agitation est utilisé, il peut s'agir d'un système d'agitation mécanique, ou magnétique. D'une manière générale, l'agitation par sonication est à éviter car celle-ci peut endommager les nanotubes de carbone. Dans un mode de réalisation particulier, le procédé est effectué sous agitation mécanique. Dans un autre mode de réalisation, le procédé est effectué sous agitation magnétique.

Selon un mode particulier de mise en oeuvre du procédé de la présente invention, on peut appliquer après l'étape (a) et avant l'étape (b), une étape (a1) de filtration. Par exemple, lorsque l'étape (a) du procédé implique une réduction en présence d'un sel de métal alcalin obtenu à partir d'un métal alcalin, la filtration peut permettre de séparer la phase liquide (par exemple une solution de K+Napht- dans du THF) de la phase solide comprenant le sel polyélectrolyte de nanotubes de carbone et éventuellement des nanotubes de carbone non réduits. Le sel polyélectrolyte de nanotubes de carbone ainsi obtenu peut être rincé une ou plusieurs fois avec un solvant adéquat. Par exemple, après l'étape de filtration (a1), le sel polyélectrolyte de nanotubes de carbone peut être rincé avec le même solvant utilisé lors de l'étape (a), notamment le THF. Le sel polyélectrolyte de nanotubes de carbone ainsi rincé peut être éventuellement séché avant l'étape (b).

Selon un mode de réalisation particulier, le procédé comprend en outre une étape de centrifugation (b1), celle-ci permettant de séparer toute fraction non dissoute de la solution de nanotubes de carbone individualisés réduits après l'étape (b). L'homme du métier saura déterminer les conditions de centrifugation adéquates pour obtenir une solution de nanotubes de carbone individualisés réduits limpide, c'est-à-dire ne comprenant pas d'agrégats détectables. Par exemple, la centrifugation peut être effectuée entre 100 et 200000 g , pendant 0.1 à 24 heures. Dans un mode de réalisation particulier, l'étape de centrifugation est effectuée à 2800 g pendant 1 heure.

Selon un mode de réalisation, la présence d'agrégats dans la solution au cours de la centrifugation est vérifiée à l'oeil nu. Ainsi, un échantillon de la solution peut être prélevé à différents intervalles dans l'étape de centrifugation pour déterminer lorsque celle-ci aura permis d'obtenir une solution limpide (c'est-à-dire sans agrégats visibles à l'oeil nu). L'examen à l'oeil nu permet de détecter des agrégats éventuels ayant une taille minimale de l'ordre du 10ième de millimètre (100 microns).

Selon un mode de réalisation, la présence d'agrégats dans la solution au cours de la centrifugation est vérifiée par microscope optique. Ainsi, un échantillon de la solution peut être prélevé à différents intervalles dans l'étape de centrifugation pour déterminer lorsque celle-ci aura permis d'obtenir une solution limpide (c'est-à-dire sans agrégats visibles au microscope optique). L'examen au microscope optique permet de détecter des agrégats éventuels ayant une taille minimale de l'ordre du micron. Dans un mode de réalisation particulier, l'échantillon de la solution peut être analysé au microscope optique avec un grossissement de 20 à 100, voire 400.

Par "nanotube", on entend, au sens de la présente description, une structure tubulaire à base de carbone, et qui possède un diamètre compris entre 0,5 et 200 nm. Ces composés appartiennent à la famille dite des "matériaux nanostructurés", qui présentent au moins une dimension caractéristique de l'ordre du nanomètre. Pour plus de détails concernant ces matériaux et leurs modes de synthèse, on pourra notamment se reporter à l'article "Nanotubes from carbon"de P.M. Ajayan (Chem. Rev., vol. 99, p.1787, 1999) [ref 9].

Le procédé de l'invention est très versatile, et a l'avantage de pouvoir être utilisé aussi bien à partir de nanotubes monoparoi que de nanotubes multiparoi, qui sont moins onéreux.

Avantageusement, on emploiera dans l'étape (a) du procédé des nanotubes mono- ou multiparoi ayant un diamètre moyen compris entre 0,5 et 100 nm.

Dans un mode de réalisation particulier, les nanotubes de carbone utilisés dans l'étape (a) du procédé sont des nanotubes monoparoi ayant un diamètre moyen compris entre 0,7 et 2,0 nm, de préférence entre 0,8 et 1,4 nm.

Dans un autre mode de réalisation particulier, les nanotubes de carbone utilisés dans l'étape (a) du procédé sont des nanotubes multiparoi ayant un diamètre moyen compris entre 2 et 20 nm, de préférence entre 10 et 15 nm.

Par ailleurs, la longueur moyenne de nanotubes employés dans l'étape (a) est en général entre 0,05 et 1000 microns.

Avantageusement, la solution de nanotubes de carbone individualisés préparée dans l'étape (b) comprend entre 0.1 et 10 g de nanotubes par litre de solution. Dans un mode de réalisation préféré, la solution de nanotubes de carbone individualisés préparée dans l'étape (b) comprend entre 0.1 et 2 g de nanotubes par litre de solution.

Selon l'invention, la structure de l'aérogel peut être obtenue par congélation lente ou brutale de la solution de nanotubes de carbone individualisés réduits obtenue à l'issue de l'étape b) du procédé.

Selon un mode particulier de réalisation, la structure de l'aérogel est obtenue par congélation brutale de la solution de nanotubes de carbone individualisés réduits obtenue à l'issue de l'étape b) du procédé. A cet effet, l'étape de congélation c) peut être réalisée en plaçant ladite solution obtenue dans l'étape (b) à une température inférieure à -50°C, ou par exemple inférieure à -80°C, ou encore par exemple inférieure à -100°C, ou encore par exemple inférieure à -150°C, ou encore par exemple inférieure à -180°C, ou encore par exemple inférieure à -190°C. Dans un mode de réalisation particulier, l'étape de congélation est effectuée de façon brutale par immersion de la solution de nanotubes de carbone individualisés dans l'azote liquide.

Dans un mode de réalisation particulier, la solution de nanotubes de carbone individualisés obtenue à l'issue de l'étape b) est une solution dans le DMSO. La congélation de la solution de nanotubes de carbone dans DMSO peut être congelée brutalement par exposition à une température très inférieure à la température de congélation du DMSO (notamment par immersion dans de l'azote liquide). La congélation de la solution de nanotubes de carbone dans DMSO peut être également obtenue de façon lente , c'est-à-dire par une méthode plus douce (ne faisant pas intervenir de congélation brutale), par exemple, par immersion dans un milieu thermostaté à une température inférieure à la température de congélation du DMSO. Par exemple, l'étape de congélation peut être réalisée par immersion de la solution de nanotubes de carbone obtenue à l'étape b) dans un bain thermostaté à une température inférieure à 18°C.

Selon un mode particulier de réalisation, la structure de l'aérogel est obtenue par congélation lente de la solution de nanotubes de carbone individualisés réduits obtenue à l'issue de l'étape b) du procédé. A cet effet, l'étape de congélation c) est avantageusement réalisée par immersion de la solution de nanotubes de carbone individualisés obtenue dans l'étape (b) dans un milieu thermostaté à une température inférieure à la température de congélation du solvant apolaire aprotique utilisé dans l'étape b).

Dans un mode de réalisation particulier, l'étape de sublimation d) est effectuée par lyophilisation à froid, avec ou sans bain thermostaté, de la solution de nanotubes congelée. Lorsqu'un bain thermostaté est utilisé, avantageusement, l'étape de sublimation est effectuée en thermostatant la solution de nanotubes de carbone individualisés à une température suffisamment inférieure à la température de congélation du solvant apolaire aprotique utilisé dans l'étape b). Dans un mode de réalisation particulier, la température du bain thermostaté est inférieure d'au moins 1°C à la température de congélation du solvant polaire aprotique, de préférence inférieure d'au moins 5°C à la température de congélation du solvant polaire aprotique.

Dans un mode de réalisation particulier, l'étape de sublimation d) est effectuée à une pression inférieure ou égale à 10⁻² mbars, de préférence inférieure ou égale à 10⁻³ mbars, de préférence inférieure ou égale à 10⁻⁴ mbars, plus avantageusement inférieure ou égale à 10⁻⁵ mbars. Dans un mode de réalisation particulier, l'étape de sublimation d) est effectuée à une pression de 10⁻⁶ mbars. Une pompe à vide puissante peut être utilisée pour obtenir la pression adéquate pour effectuer la sublimation. Un lyophilisateur, conçu pour des solutions aqueuses, ne suffit pas pour obtenir l'aérogel. Par exemple, un contrôle judicieux de paramètres comme la température de réfrigération (choix du bain réfrigérant pour garder la solution organique congelée), et le vide appliqué (choix d'une pompe à vide adéquate pour obtenir un vide satisfaisant, nécessité de s'affranchir des connexions en caoutchouc souvent utilisées dans les lyophilisateurs (i.e., utilisation d'une connexion « directe » entre l'enceinte contenant la solution à lyophiliser et la pompe à vide), et de s'assurer que le volume de l'enceinte sous vide ne soit pas trop important par rapport au volume de solution à évaporer) est nécessaire. Dans un mode de réalisation, la lyophilisation est effectuée avec une pompe à vide susceptible de générer une pression inférieure à 0,1 mbars. Selon la nature du solvant, en particulier sa tension de vapeur, une pompe turbomoléculaire peut être utilisée.

En fonction des besoins, l'aérogel peut être mis en forme avant ou après la sublimation (ou lyophilisation). Par exemple, l'aérogel peut être « moulé » en choisissant la forme appropriée du récipient dans lequel la solution congelée est sublimée (ou lyophilisée). L'aérogel peut également être mis sous forme de fines membranes après sublimation (ou lyophilisation) par compression de l'aérogel.

Dans un mode de réalisation particulier, l'étape de sublimation d) est mise en oeuvre avec une pompe suffisamment puissante pour obtenir, au-dessus de la solution congelée, une pression inférieure à la pression de vapeur saturante du solvant. Par ailleurs, afin d'optimiser le vide, le dispositif de sublimation peut être conçu de façon à ce qu'il y ait une liaison directe entre le tube à lyophiliser et la pompe. Un tel dispositif permet d'éviter l'utilisation de tuyaux en caoutchouc qui présente l'inconvénient de « dégazer » et ne permettent pas d'obtenir le vide requis pour la sublimation, en particulier pour les solvants organiques polaires tels que le DMSO. Par ailleurs, cette liaison directe permet un passage de l'enceinte sous atmosphère inerte utilisée pour la préparation de la solution de nanotubes de carbone à la pompe à vide, sans exposition de la solution à l'air (ce qui conduirait à l'oxydation des nanotubes, et donc à leur réaggrégation).

Dans un mode de réalisation particulier, le solvant polaire aprotique utilisé dans l'étape b) est le DMSO et l'étape de sublimation est effectuée en thermostatant la solution de nanotubes de carbone dans le DMSO à une température inférieure à 18°C (température de congélation du DMSO), par exemple 6°C. Dans ce mode de réalisation particulier, l'étape de sublimation d) est effectuée à une pression inférieure ou égale à 10⁻² mbars, de préférence inférieure ou égale à 10⁻³ mbars, de préférence inférieure ou égale à 10⁻⁴ mbars, plus avantageusement inférieure ou égale à 10⁻⁵ mbars. Dans un mode de réalisation particulier, le solvant polaire aprotique est le DMSO et l'étape de sublimation d) est effectuée à une pression de 10⁻⁵ mbars.

Selon un autre aspect, la présente invention fournit également des aérogels de nanotubes de carbone susceptibles d'être obtenus par un procédé selon l'invention. Par exemple, les aérogels peuvent être utilisés comme tels, ou peuvent être déposés sur un substrat ou mélangés à un autre matériau.

A l'issue du procédé de l'invention, on obtient un matériau à base de nanotubes de carbones individualisés, généralement monolithique. Le matériau obtenu a la forme de la solution congelée, mais il ne reste qu'un réseau percolé (en contact) de nanotubes de carbones individualisés.

Les aérogels de l'invention présentent par ailleurs une très faible densité apparente, en particulier bien inférieure à celles des aérogels de nanotubes carbone actuellement connus. Par exemple, Yodh *et al.* [ref 16] rapportent des aérogels de densité comprise entre 10 et 30 mg/cm³. Backov *et al.* [ref 17] rapportent des densités apparentes "typiquement aux alentours de 0.2 g/cc" (c'est-à-dire 200 mg/cm³). Ainsi, généralement, un aérogel tel qu'obtenu selon l'invention a une densité apparente le plus souvent inférieure ou égale à 10 mg/cm³, généralement inférieure ou égale à 5 mg/cm³, de préférence 2 ± 0,5 mg /cm³. Un aérogel tel qu'obtenu selon l'invention a une densité apparente comprise entre 0.1 et 10 mg/cm³, généralement comprise entre 0.1 et 5 mg/cm³, de préférence comprise entre 0.1 et 2 ± 0,5 mg /cm³.

Cette très faible densité apparente traduit un volume poreux très élevé pour les aérogels de l'invention, où, le plus souvent, le volume occupé par les pores représente au moins 99% du volume total de l'aérogel, généralement au moins 99,5%, voire au moins 99,8%. Comme il sera apparent à l'homme du métier, le volume poreux peut être influencé par la concentration de la solution de nanotubes de carbone individualisés obtenue à l'étape b). On pourra donc augmenter le volume poreux de l'aérogel en utilisant une solution de nanotubes de carbone individualisés de plus faible concentration. On appréciera cependant que la tenue mécanique de l'aérogel final sera vraisemblablement moindre. Il conviendra donc d'adapter la concentration de la solution de nanotubes de carbone individualisés en fonction de l'application à laquelle l'aérogel est destiné.

Généralement, la concentration de la solution de nanotubes de carbone individualisés est comprise entre 0.01 et 10 mg/g ou entre 0.001 et 1 % v/v, avantageusement entre 0.1 et 4 mg/g ou entre 0.01 et 0.4 % v/v, plus avantageusement entre 1 et 2 mg/g ou entre 0.1 et 0.2 % v/v. Généralement, la concentration de la solution de nanotubes de carbone individualisés est de l'ordre de 1 mg/g ou 0,1% v/v.

De façon générale, les aérogels de carbone ainsi obtenus présentent une structure de grande porosité. Il s'agit de porosités ouverte et fermée, cette dernière pouvant être considérée comme négligeable. On entend par matériau à porosité ouverte un matériau dans lequel l'ensemble des pores communique avec la surface du matériau. La porosité fermée correspond au volume intérieur des nanotubes de carbone.

Les aérogels obtenus selon le procédé de l'invention présentent également une surface spécifique supérieure à celle des aérogels de nanotube de carbone connus jusqu'à présent (due au fait que, dans les présents aérogels, les nanotubes de carbones sont individualisés). De ce fait, les aérogels de l'invention ont le plus souvent une surface spécifique élevée, en général comprise entre 100 et 2000 m²/g, cette surface spécifique étant avantageusement supérieure à 200 m²/g, de préférence supérieure à 250m²/g, de préférence supérieure à 300 m²/g Au sens de la présente description, le terme de "surface spécifique " se réfère à la surface spécifique BET, telle que déterminée par adsorption d'azote, selon la méthode bien connue dite de BRUNAUER - EMMET - TELLER qui est décrite dans The journal of the American Chemical Society, volume 60, page 309 (1938), et correspondant à la norme internationale ISO 5794/1.

Une surface spécifique aussi élevée que 2000 m²/g peut être observée notamment si une certaine proportion des tubes sont ouverts.

Par ailleurs, le caractère monolithe (d'un seul tenant) de l'aérogel, outre qu'il assure un contact électrique, permet une certaine tenue mécanique.

Cette structure particulière des aérogels de l'invention peut notamment être mise en évidence sur des clichés des matériaux obtenus par microscopie à balayage à effet de champ, dont des exemples sont donnés sur les Figures ci-annexées. On y remarque en particulier deux types de porosité ouverte : des pores en volume de diamètre de l'ordre de 20 microns et des pores bidimensionnels (trous dans les parois des premiers pores) de diamètre de 10 à 100 nanomètres.

Les aérogels obtenus selon la présente invention sont électriquement conducteurs. A ce titre, ils pourraient avantageusement être utilisés pour la préparation de composants électrochimiques. Par exemple, ils pourraient remplacer les électrodes de carbone dans les piles à combustible, les biocapteurs et/ou les supercondensateurs, du fait de leur grande surface spécifique. La porosité accrue par rapport à des électrodes de carbone classiques devrait donner des saut quantitatifs en termes de performance pour les applications énergétiques (piles à combustible, biocapteurs, supercondensateurs).

La structure particulière des aérogels de carbone obtenus selon l'invention les rend particulièrement adaptés à titre de matériaux de séparation, notamment pour effectuer des séparations de type liquide/liquide. En particulier, les aérogels de la présente invention sont adaptés pour l'adsorption de molécules hydrophobes, notamment présentes dans les milieux aqueux. Ainsi, une application des aérogels de la présente concerne la décontamination des milieux aqueux contenant des polluants hydrophobes tels que les hydrocarbures, par exemple.

Dans ce cadre, la grande porosité de l'aérogel permet d'obtenir une bonne diffusion des espèces, induisant ainsi des vitesses de séparation élevée. Dans ce cadre, les aérogels de l'invention peuvent par exemple être employés pour la constitution de membranes ou de matériaux de filtration, notamment pour la filtration ou bien pour le nettoyage d'eaux usées.

A ce titre, l'enseignement de la demande de brevet français N° FR 2 881 362 [ref 22] pourra être adapté pour mettre en oeuvre des dispositifs de dépollution utilisant les aérogels de l'invention. La différence notoire des présent aérogels avec les matériaux nanostructurés à base de carbone de la demande de brevet précitée est leur densité apparente inégalée. A ce titre, les aérogels de la présente demande représentent donc une alternative plus avantageuse et plus efficace aux matériaux décrits dans la demande FR 2 881 362 pour retenir les espèces hydrophobes de milieux aqueux.

Les aérogels peuvent être mis en oeuvre sous forme de membranes par compression dudit aérogel. Ils peuvent donc être utilisés comme membranes de filtration et/ou dépollution, notamment pour la dépollution des nappes de pétrole. La grande surface spécifique des aérogels selon l'invention, ainsi que leur hydrophobicité, permet l'adsorption préférentielle d'huile dans un mélange huile/eau. Ainsi, lesdits aérogels permettent d'envisager des solutions aux problèmes de pollution, en particulier pour le traitement des eaux ou de marées noires, par adsorption des hydrocarbures présents en milieu aqueux, alors qu'il n'existe pas à l'heure actuelle de moyen performant pour résoudre ces problèmes de pollution.

Compte tenu de leur structure spécifique, les aérogels de l'invention sont également bien adaptés à d'autres applications. En particulier, du fait du caractère biocompatible du carbone, ils peuvent notamment être employés pour la préparation de biomatériaux, et en particulier pour la préparation de support pour la croissance cellulaire, pour la croissance osseuse ou pour le remplacement de cartilage. Ils peuvent notamment trouver application pour la préparation de supports biocompatibles pour la croissance de cellules osseuses, ou de neurones. Dans ce type d'application, la porosité particulière du matériau assure une colonisation optimale : la porosité assure d'une part une diffusion des cellules, qui peuvent accéder sensiblement à l'ensemble de la surface du matériau, et d'autre part une irrégularité de la surface du matériau propre à assurer une bonne fixation des cellules sur l'aérogel.

Dans certains modes de réalisation, afin d'assurer une topologie optimale pour la croissance cellulaire, la solution de nanotubes de carbone individualisés réduits obtenue à l'étape (b) peut être remplie de billes insolubles dans le solvant polaire aprotique considéré. Après lyophilisation, l'aérogel se forme autour de ces billes. Ainsi, le procédé de l'invention peut comprendre en outre une étape (b1) d'addition de billes de matériau insoluble dans le solvant polaire aprotique de l'étape (b). Lesdites billes peuvent avoir un diamètre compris entre 10 microns et 1 mm, de préférence entre 50 et 100 microns.

Les billes peuvent être ensuite éliminées par dissolution ou attaque acide, laissant un aérogel à pores contrôlés.L'utilisation de telles particules sphériques comme "templates" est très répandue dans la littérature scientifique et l'homme de l'art peut facilement appliquer et/ou adapter cette technique aux solutions de nanotubes de carbone réduits décrites ici.

Les aérogels peuvent être mis en oeuvre sous forme de membranes par compression dudit aérogel. Le caractère élastique de ces membranes (dû à la présence de pores), ainsi que leur excellente résistance à l'usure et leur biocompatibilité, font de ces matériaux des candidats particulièrement intéressants pour des applications dans le domaine du remplacement de cartilage.

Il faut noter que les aérogels de l'art antérieur étant préparés à partir de suspensions aqueuses de nanotubes de carbone en présence de tensioactifs non-biocompatibles (cf. travaux de Yodh et al. et Backov et al.), ils ne sont pas vraiment adaptés aux applications biomédicales. Même si Yodh *et al.* décrivent une étape d'élimination du tensioactif utilisé, il reste une incertitude quant à la présence éventuelle de résidus de tensioactifs. De plus, cela requiert une étape d'élimination des tensioactifs supplémentaire qui n'est pas forcément facile à mettre en oeuvre, ni même avantageux sur le plan industriel. Les aérogels de la présente invention présentent donc un avantage technique non négligeable pour l'élaboration de nouveaux matériaux biocompatibles.

Plus généralement les aérogels de l'invention peuvent être employés dans la plupart des applications connues des aérogels à base de carbone, dans la mesure où ils présentent les avantages spécifiques de ces matériaux, notamment une grande inertie chimique, notamment vis-à-vis des réducteurs, une stabilité thermique importante jusqu'à plus de 2000°C (en milieu non oxydant), ainsi qu'une très bonne conductivité thermique et électrique.

Les aérogels de l'invention peuvent ainsi, en particulier, être utilisés pour la préparation de support d'espèces catalytiques, par exemple pour la catalyse de réactions en milieu réducteur, notamment à haute température. Ainsi, pour certaines applications, les aérogels obtenus peuvent être post-traités, par exemple pour être imprégnés par des espèces catalytiques. Dans un mode de réalisation particulier, les aérogels sont utilisés pour la préparation de supports de catalyseur pour la catalyse hétérogène.

Imprégnés par exemple par du silicium, des résines ou des polymères, les aérogels peuvent également permettre d'obtenir des composites conducteurs, du fait de leur réseau percolé (en contact). Ainsi, la présente invention concerne également l'utilisation des aérogels susceptibles d'être obtenus par le procédé de l'invention pour la préparation de matériaux composites.

Par "imprégné", on entend ici une imprégnation de l'aérogel avec un matériau soit:
(i) en imbibant l'aérogel avec un autre matériau, sous forme liquide ou en solution, pénétrant dans la porosité de l'aérogel. Ainsi, selon ce mode de réalisation particulier, le procédé de l'invention comprend en outre une étape e) consistant à imbiber ledit aérogel avec un autre matériau, sous forme liquide ou en solution. Il peut s'agir par exemple d'un polymère, un mélange de polymères ou une résine, sous forme liquide ou en solution, ou du silicium en fusion. L'aérogel se trouve ainsi imbibé dudit matériau sous forme liquide ou en solution; soit
(ii) par mélange d'une solution d'un matériau avec la solution de nanotube de carbone individualisés obtenue à l'issue de l'étape b) du procédé de l'invention. Le matériau peut être par exemple un polymère, un mélange de polymères, ou une résine. Ainsi, selon ce mode de réalisation particulier, le procédé de l'invention comprend en outre, avant l'étape de congélation c), une étape b2) de mélange d'une solution d'un matériau (par exemple une solution de polymère, d'un mélange de polymères, ou d'une résine) avec la solution de nanotube de carbone individualisés réduits obtenue à l'étape b). Le mélange est ensuite congelé et sublimé selon les étapes c) et d) du procédé pour former un aérogel composite.

Les résines utilisées peuvent être des résines polyesters insaturées (utilisées par exemple dans les plastiques renforcés de fibre de verre), les résines époxydes (utilisées par exemple dans les adhésifs et dans la fabrication des plastiques), les résines phénoliques ou les résines polyimides.

Selon l'invention, le polymère peut être tout polymère permettant de mettre en oeuvre la présente invention. Il peut être choisi par exemple dans le groupe comprenant le polystyrène ; les polyoléfines, par exemple le polyéthylène, le polypropylène, les poly(alpha-oléfine)s, le polyisobutène et le polypropylène ; les polyéthers ; les polyesters ; les polyamides ; les polyacrylamides ; les polyacrylates (par exemple le polymethyl methacrylate ou « PMMA ») ; les polysilanes ; les polysiloxanes.

Selon l'invention, le polymère peut être un copolymère à blocs linéaire ou un copolymère statistique. L'homme du métier saura identifier des conditions opératoires adéquates et le ou les polymères à utiliser pour réaliser un matériau composite ayant les propriétés requises/désirées. Notamment, l'homme du métier pourra s'inspirer des méthodes décrites dans FR 04/05120 [ref 29] et/ou WO 2006/136715 [ref 30] qui décrivent la préparation de matériaux composites à partir de nanotubes de carbone et de polymères ou mélanges de polymères. L'homme du métier saura adapter les méthodes décrites dans ces documents pour réaliser la préparation d'aérogels composites à partir d'aérogels susceptibles d'être obtenus par le procédé de la présente demande. Le (ou les) polymère(s) peu(ven)t être sélectionné(s) de façon à optimiser les interactions de surface polymère/nanotubes de carbone.

Par « copolymère à blocs », on entend dans la présente un polymère séquencé comprenant plus d'une espèce de monomère. Dans un copolymère à blocs, des monomères identiques sont groupés. De tels polymères et leur procédé de fabrication sont décrits par exemple dans Matyjaszewski, K. ; Eds. ; Advances in Controlled/Living Radical Polymerization, (American Chemical Society 2003) [ref 23] ou Hsieh, H. L. ; Quirk, R. P. ; Eds. ; Anionic Polymerization Principles and Practical Applications, (Marcel Dekker 1996) [ref 24].

Par « copolymère statistique », on entend dans la présente un polymère dans lequel les différents monomères se mélangent en fonction de la réactivité et de la concentration de ceux-ci. De tels polymères et leurs procédés de fabrication sont décrits par exemple dans Matyjaszewski, K.; Davies, T. P; Eds.; Handbook of Radical Polymerization, (Wiley-Interscience 2002) [ref 25] ou Fontaine, L. ; Initiation à la Chimie et à la Physico-Chimie Macromoléculaires (Groupe Français d'Etudes et d'Applications des Polymères volume 12 (Chapitre 3)) [ref 26].

Selon l'invention, lorsqu'il s'agit d'un copolymère à blocs, il peut s'agir par exemple d'un copolymère diblocs synthétisé par exemple par polymérisation radicalaire contrôlée ou par polymérisation anionique vivante ou par polymérisation cationique vivante ou un copolymère statistique synthétisé par polymérisation radicalaire contrôlée ou polymérisation radicalaire non contrôlée.

La polymérisation radicalaire contrôlée (PRC) est une méthode de choix pour préparer des polymères et copolymères bien définis avec des masses molaires modulables et des indices de polymolécularité faibles. Des techniques utilisables dans la présente invention sont décrites par exemple dans Matyjaszewski, K.; Davies, T. P; Eds.; Handbook of Radical Polymerization, (Wiley-Interscience 2002) [ref 25].

On entend par « polymérisation vivante » une polymérisation dans laquelle il n'y a ni réactions de terminaison, ni réactions de transfert, et où les chaînes de polymères continuent à croître tant qu'il reste des molécules de monomères à ajouter aux chaînes. Selon l'invention, la polymérisation vivante peut être cationique ou anionique. De tels procédés sont décrits par exemple dans Matyjaszewski, K. ; Eds. ; Cationic Polymerizations Mechanisms, Synthesis, and Applications, (Marcel Dekker 1996) [ref 31] ou Hsieh, H. L. ; Quirk, R. P. ; Eds. ; Anionic Polymerization Principles and Practical Applications, (Marcel Dekker 1996) [ref 24].

Les monomères peuvent être introduits en totalité pendant l'étape de polymérisation. Ils peuvent également être introduits séparément ou en mélange, en continu ou en discontinu. Un supplément de monomère peut également être introduit en fin de polymérisation afin d'obtenir la composition souhaitée en polymère.

Les adjuvants éventuellement incorporés lors des procédés de polymérisation classiques sont utilisables selon le procédé de l'invention. Ainsi, on peut utiliser des initiateurs, des agents de transfert de chaînes, des catalyseurs, des antioxydants et des lubrifiants connus de l'homme du métier.

### (i) Imprégnation en imbibant l'aérogel d'un autre matériau

Selon un mode de réalisation, le matériau composite est obtenu par imprégnation d'un aérogel obtenu selon l'invention avec un autre matériau, sous forme liquide ou en solution, par exemple du silicium, un polymère ou mélange de polymères, ou une résine.

Par "imprégnation", on entend ici une imprégnation de l'aérogel en imbibant celui-ci selon le mode de réalisation (i) ci-dessus.

Ainsi, dans un mode de réalisation particulier, le procédé comprend en outre une étape supplémentaire e) qui consiste à imbiber l'aérogel de l'invention avec un autre matériau (par exemple un polymère, un mélange de polymères ou une résine, sous forme liquide ou une solution de ceux-ci, ou du silicium en fusion), ledit matériau étant sous forme liquide ou en solution. Par exemple, l'aérogel peut être imbibé de PMMA ou d'une solution de PMMA. Par exemple, le PMMA peut être en solution dans le même solvant que les nanotubes réduits de l'étape b). Par exemple ce solvant peut être le sulfolane, le diméthylsulfoxyde (DMSO), le diméthylformamide, la N-méthylpyrrolidone ou le N-méthylformamide. Dans un mode de réalisation particulier, le solvant est le DMSO. Dans un autre mode de réalisation particulier, le solvant est la N-méthylpyrrolidone.

Ladite étape e) peut être suivie d'une étape de solidification de l'aérogel composite, par séchage, pour éliminer le solvant, par exemple en chauffant l'aérogel imprégné, éventuellement sous pression réduite, de manière à évaporer le solvant des pores de l'aérogel.

En ce qui concerne l'imprégnation des aérogels de l'invention par le silicium, celle-ci peut permettre d'une part la densification de l'aérogel, et d'autre part la combinaison des propriétés du carbure de silicium (dureté et stabilité à haute température) avec la porosité de l'aérogel.

Ainsi, dans un mode de réalisation particulier, l'aérogel de l'invention peut être imprégné de silicium en fusion. L'aérogel étant composé de nanotubes de carbone, cette imprégnation de silicium est dans ce cas une imprégnation réactive : le silicium réagit alors avec le carbone pour former du carbure de silicium.

Par "imprégnation de silicium ", on entend ici une imprégnation de l'aérogel par une phase de type silicium en fusion pénétrant dans la porosité de l'aérogel.

Le silicium en fusion est très fluide et possède un fort pouvoir mouillant notamment vis-à-vis de surfaces en carbone. Ainsi, lorsqu'un aérogel selon l'invention est imprégné par du silicium à l'état liquide, celui-ci progresse dans le réseau de porosités du matériau en suivant la surface des pores.

Dans un autre mode de réalisation particulier, l'aérogel peut être imprégné par une composition contenant un composé organosilicié en solution, par exemple un composé organosilicié précurseur du carbure de silicium tel qu'un polycarbosilane.

Dans un autre mode de réalisation particulier, l'aérogel de l'invention peut être imprégné par du silicium et/ou du germanium (c'est-à-dire du silicium seul, du germanium seul ou un mélange de silicium et de germanium en toutes proportions).

Dans un autre mode de réalisation particulier, l'aérogel de l'invention peut être imprégné majoritairement par du silicium et/ou du germanium allié avec au moins un métal ou un autre métalloïde. Dans ce dernier cas, le métal ou autre métalloïde peut être choisi notamment parmi le fer, le cobalt, le titane, le zirconium, le molybdène, le vanadium, le carbone ou le bore selon les propriétés particulières à conférer à l'aérogel après imprégnation de silicium.

L'homme du métier saura identifier des conditions opératoires adéquates pour mettre en oeuvre un procédé d'imprégnation de l'aérogel de l'invention par du silicium et/ou du germanium, éventuellement allié avec au moins un métal ou un autre métalloïde. Par exemple, on pourra se référer à la demande de brevet internationale publiée sous le N° WO 2004/076381 [ref 36].

### (ii) Imprégnation par mélange d'une solution d'un matériau avec la solution de nanotube de carbone individualisés obtenue à l'issue de l'étape b)

Selon un autre mode de réalisation, le matériau composite est obtenu par un procédé comprenant une étape b2) de mélange d'une solution d'un matériau avec la solution de nanotube de carbone individualisés obtenue à l'étape b). Ledit matériau peut être par exemple un polymère, un mélange de polymères, ou une résine. Le matériau peut être en solution dans le même solvant que celui utilisé pour former la solution de nanotube de carbone individualisés obtenue à l'étape b). Par exemple, il peut s'agir du sulfolane, du diméthylsulfoxyde, du diméthylformamide, de la N-méthylpyrrolidone ou du N-méthylformamide. Dans un mode de réalisation particulier, le solvant est le DMSO. Dans un autre mode de réalisation particulier, le solvant est la N-méthylpyrrolidone.

Ainsi, selon ce mode de réalisation particulier, le procédé de l'invention comprend en outre, avant l'étape de congélation c), une étape b2) de mélange d'une solution d'un matériau avec la solution de nanotube de carbone individualisés obtenue à l'étape b). Le mélange est ensuite congelé et sublimé selon les étapes c) et d) du procédé pour former un aérogel composite. Il est entendu que les différents modes de réalisation des étapes c) et d) décrits dans la présente sont également applicables à la mise en oeuvre d'un aérogel composite selon le mode de réalisation (ii) décrit ci-dessus.

Plusieurs variantes du mode de réalisation (ii) peuvent être mises en oeuvre.

Ainsi, selon un mode de réalisation, le procédé selon le mode de réalisation (ii) comprend une étape de polymérisation *in situ* d'un monomère ou mélange de monomères dans ladite solution de nanotube de carbone individualisés réduits. De tels polymères et leur procédé de fabrication sont décrits par exemple dans Matyjaszewski, K. ; Eds. ; Advances in Controlled/Living Radical Polymerization, (American Chemical Society 2003) [ref 23]; Hsieh, H. L. ; Quirk, R. P. ; Eds. ; Anionic Polymerization Principles and Practical Applications, (Marcel Dekker 1996) [ref 24] ; Matyjaszewski, K.; Davies, T. P; Eds.; Handbook of Radical Polymerization, (Wiley-Interscience 2002) [ref 25] ou Fontaine, L. ; Initiation à la Chimie et à la Physico-Chimie Macromoléculaires (Groupe Français d'Etudes et d'Applications des Polymères volume 12 (Chapitre 3)) [ref 26].

Selon un mode de réalisation, le procédé selon le mode de réalisation (ii) comprend une étape de polymérisation-greffage d'un ou plusieurs monomères sur un ou plusieurs nanotubes de carbone individualisés. Des méthodes de polymérisation-greffage sont bien connues dans l'art. L'homme du métier saura identifier des conditions opératoires adéquates pour mettre en oeuvre un procédé de polymérisation-greffage d'un monomère sur un ou plusieurs nanotubes de carbone individualisés.

Selon un mode de réalisation particulier, les nanotubes de carbone individualisés sont fonctionnalisés par une ou plusieurs greffes de groupements fonctionnels avant leur association avec le(s) polymère(s). Dans ce contexte, on entend par « association » la combinaison des nanotubes de carbone greffés avec le(s) polymère(s) par simple mélange, par polymérisation *in situ* d'un monomère ou mélange de monomères dans une solution de nanotubes de carbone greffés, ou par polymérisation-greffage d'un ou plusieurs monomères sur un ou plusieurs nanotubes de carbone greffés. La fixation desdits groupements fonctionnels sur les nanotubes de carbone peut être réalisée par tout procédé approprié de chimie organique connu de l'homme du métier. On trouvera par exemple un panorama général des méthodes de fonctionnalisation des nanotubes de carbone dans: "Chemistry of Carbon Nanotubes" de Dimitrios Tasis, Nikos Tagmatarchis, Alberto Bianco et Maurizio Prato, Chem. Rev. 2006, 106, 1105-1136 [ref 33]. Il peut s'agir par exemple de greffes de groupes polyéthylène glycol, ou de groupes acide. Ces greffes peuvent (i) permettre une fonctionnalisation des nanotubes de carbone individualisés, (ii) augmenter les interactions entres les nanotubes de carbone individualisés (Liaisons de type Van der Waals, liaisons hydrophobes ou liaisons hydrogène), et/ou (iii) augmenter les interactions entre les nanotubes de carbone individualisés et le(s) polymère(s) au(x)quel(s) ils sont associés, et peuvent présenter l'avantage de renforcer les aérogels composites comprenant ces nanotubes de carbone individualisés.

Selon un mode de réalisation particulier, le matériau composite est obtenu à partir d'une solution de nanotube de carbone individualisés et d'une solution de PMMA, par exemple dans du DMSO. Ainsi, selon ce mode de réalisation particulier, un aérogel composite PMMA/nanotubes de carbone peut être obtenu en introduisant, avant l'étape de congélation c), une étape de mélange d'une solution de PMMA (par exemple dans du DMSO) avec la solution de nanotube de carbone individualisés réduits obtenue à l'étape b). Le mélange est ensuite congelé et sublimé pour former un aérogel composite PMMA/nanotubes de carbone.

Les aérogels composites selon l'invention peuvent être utilisés dans l'ensemble des applications prévues pour les aérogels de l'invention en leur conférant une meilleure tenue mécanique. Par exemple, ces aérogels composites peuvent être utilisés comme matériaux de séparation (e.g., membranes de filtration, membranes de dépollution à étendre en surface aqueuse pour absorber des hydrocarbures), comme supports de catalyse hétérogène, ou comme biomatériaux (dans le cas de polymères biocompatibles).

Selon un mode de réalisation particulier de l'utilisation du procédé de l'invention pour la préparation d'aérogels composites, l'aérogel composite peut comprendre, en plus des nanotubes de carbone, d'autres matériaux conventionnellement utilisés dans les matériaux composites.

Les charges entrant dans la composition desdits aérogels composites, peuvent être de dimension nanométrique et/ou micrométrique.

On entend dans la présente demande par « matériau de dimension nanométrique » un matériau dont la taille est de quelques nanomètres dans au moins une des dimensions de l'espace. Par exemple, la taille du matériau dans au moins une des dimensions de l'espace est comprise entre 1 et 20 nm, de préférence entre 1 et 2 nm.

On entend dans la présente demande par « matériau de dimension micrométrique » un matériau de taille comprise entre 1 et 100 microns.

Le matériau composite peut comprendre des charges de dimension micrométrique uniquement, ou nanométrique uniquement, ou un mélange de charges micro- et nanométriques (voir par exemple FR 2 873 381 [ref 32] où des matériaux composites comprenant des charges nano- et des charges micrométriques dans le même matériau sont décrits).

Ces différentes utilisations des aérogels de l'invention constituent encore un objet particulier de l'invention.

De façon inattendue, les aérogels de nanotubes de carbone de l'invention et leurs avantages spécifiques sont obtenus très simplement, par la succession d'étapes (a) à (d) du procédé de l'invention.

Le procédé de l'invention présente, entre autres, l'avantage d'être simple à mettre en oeuvre et peu onéreux.

Différentes caractéristiques et divers modes de réalisation avantageux du procédé de l'invention vont maintenant être décrits plus en détail.

Le procédé décrit dans la présente résout les problèmes majeurs actuels au développement d'aérogels de nanotubes de carbone. D'une part, le procédé de la présente invention implique la lyophilisation d'une solution de nanotubes de carbones dans un solvant polaire aprotique. Un avantage de ce procédé par rapport aux procédés de l'art antérieur qui sont basés sur la lyophilisation de dispersions aqueuses, est que l'utilisation d'un solvant organique permet une meilleure homogénéité de la solution à lyophiliser et une plus grande flexibilité quant à l'incorporation de constituants complémentaires, par exemple un polymère ou mélange de polymères en vue de préparer un matériau composite.

D'autre part, le procédé de la présente invention ne nécessite pas l'utilisation de tensioactifs. Il ne nécessite pas non plus l'utilisation de la sonication, qui endommage les nanotubes de carbone ou tout du moins les raccourcit. Ainsi, le rapport longueur/diamètre des nanotubes de carbone dans l'aérogel obtenu est maximal.

En effet, comme en atteste de nombreuses publications, il est largement connu que le traitement aux ultrasons (sonication) affecte l'intégrité structurale des nanotubes de carbone. En particulier, la sonication peut provoquer la coupure des nanotubes de carbone, et l'apparition de défauts tels que des courbures (« buckling », « bending ») et/ou des dislocations dans les structures carbonées.

La sonication provoque également l'arrachement des couches supérieures de feuillets de graphite, dans le cas des tubes multiparois, ce qui conduit à un amincissement des nanotubes d'une manière similaire à l'endommagement rapporté par l'oxydation. On pourra se référer par exemple, à Lago et al., « Mechanical damage of carbon nanotubes by ultrasound », Carbon, 34(6) 814-816, (1996) [ref 37] ; Badaire et al., « In situ measurement of nanotube dimensions in suspensions by depolarized dynamic light scattering », Langmuir, 20 : 10367-10370 (2004) [ref 38]; Heller et al., « Concomittant length and diameter séparation of single-walled carbon nanotubes », J. Am. Chem. Soc., 126 :14567-14573 (2004) [ref 39] ; Hennrich et al., « The mechanism of cavitation-induced scission of single-walled carbon nanotubes », J. Phys. Chem. B, 111 : 1932-1937 (2007) [ref 40].

La sonication peut également entraîner une altération des performances, notamment électriques, des nanotubes de carbone, due à l'endommagement structural précité. Voir par exemple Badaire *et al.* [ref 38].

Les travaux de Yodh et al. [ref 16] et de Backov et al. [ref 17] concernent l'obtention d'aérogels par lyophilisation ou séchage critique (« critical-point drying ») d'une suspension aqueuse de nanotubes de carbone contenant des surfactants, préalablement soumise à un traitement aux ultrasons, un procédé mécanique qui altère la structure des nanotubes de carbone, et leurs propriétés électriques.

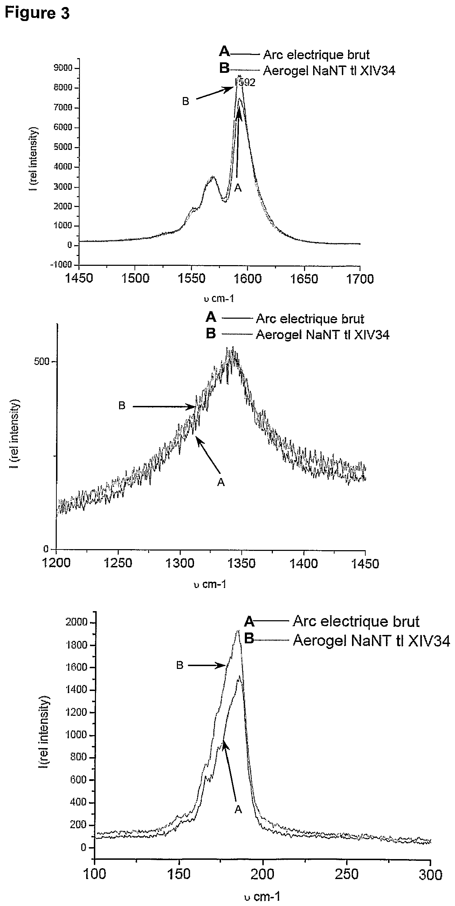

Par opposition, dans le procédé de l'invention, les nanotubes de carbone sont individualisés par une méthode chimique dite « de dissolution douce » consistant à réduire les nanotubes de carbone par un métal alcalin et dissoudre le sel polyélectrolyte obtenu dans un solvant polaire aprotique. Ainsi, le procédé de l'invention respecte la structure des nanotubes de carbone que l'on retrouve non-altérée dans l'aérogel. Voir par exemple la figure 3 qui illustre des spectres Raman de nanotubes de carbone bruts et d'un aérogel de la présente invention. Comme le montre la figure 3, les deux courbes (nanotubes bruts et aérogel) sont indifférenciables. En particulier, la bande "D" à 1300 cm⁻¹, signature du désordre et la bande "RBM" vers 200 cm⁻¹ sensible à l'environnement du tube, sont indifférenciables. Cette observation a été vérifiée à deux longueurs d'ondes différentes du laser Raman.

Par ailleurs, la sonication provoque la fragmentation des nanotubes de carbone, ce qui implique la présence de débris et particules de carbone dans le produit final. Cet inconvénient ne se retrouve pas dans les aérogels de l'invention.

Ainsi, les aérogels de la présente invention sont supérieurs aux aérogels de l'art antérieur en ce qu'ils ne présentent pas les inconvénients précités dus au processus de sonication.

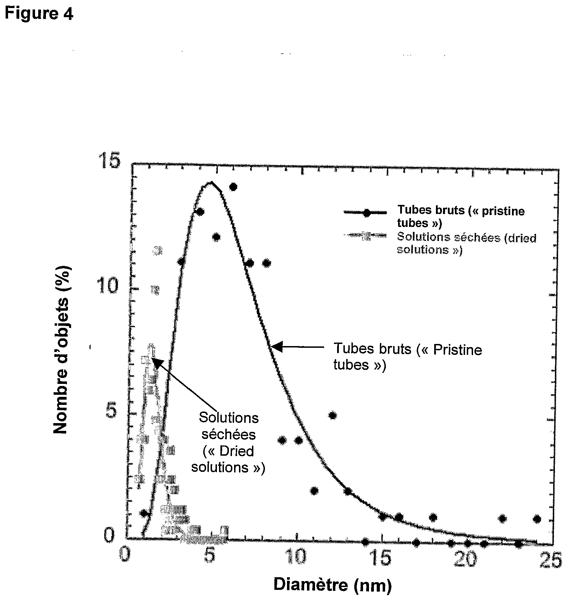

Par ailleurs, un avantage du procédé de l'invention par rapport aux procédés de l'art antérieur qui sont basés sur la lyophilisation de dispersions en présence de tensioactifs, est que la dissolution des nanotubes d'un solvant organique polaire aprotique permet une meilleure homogénéité de la solution à lyophiliser. En effet, comme le montre la figure 4, après dissolution selon le procédé de l'invention, les tubes sont tous exfoliés au contraire des tubes bruts dispersés dans l'éthanol, qui restent en gros faisceaux. Ainsi, dans un aérogel obtenu par dispersion et tensio-actifs, on a une grande distribution de taille des nanotubes si l'on sonique peu et de très petites longueurs si l'on sonique beaucoup. Voir article de Islam et al., « High weight fraction surfactant solubilization of single-wall carbon nanotubes in water », Nanoletters, Vol. 3(2) : 269-273 (2003) [ref 41].

Ainsi, le procédé de l'invention permet d'obtenir un aérogel à partir de nanotubes de carbones individualisés, c'est-à-dire par exfoliation complète des faisceaux de nanotubes.

D'autre part, les aérogels selon l'invention sont biocompatibles. Ainsi, outre les applications conventionelles des nanotubes de carbone (matériaux composites, composants électroniques, etc.), les présents aérogels peuvent être utilisés comme biomatériaux dans des applications biomédicales, par exemple comme support pour la croissance de cellules, notamment osseuses.

En outre, le procédé de l'invention permet de préparer des aérogels de nanotubes de carbone biocompatibles, ce qui est extrêmement avantageux pour toutes les applications biologiques.

Enfin, le procédé fonctionne aussi bien avec des nanotubes de carbone monoparoi que des nanotubes multiparoi.

Comme l'homme du métier peut le constater à la lecture de la présente description, un des principaux avantages de la présente invention est la possibilité d'obtenir un matériau biocompatible, particulièrement adapté pour la croissance de cellules. D'autres avantages incluent la simplicité de mise en oeuvre du procédé, ainsi que son habilité à fournir, des aérogels de nanotubes de carbone de très faible densité apparente.

D'autres avantages pourront encore apparaître à l'homme du métier à la lecture des exemples ci-dessous, illustrés par les figures annexées, donnés à titre illustratif.

### Brève description des figures

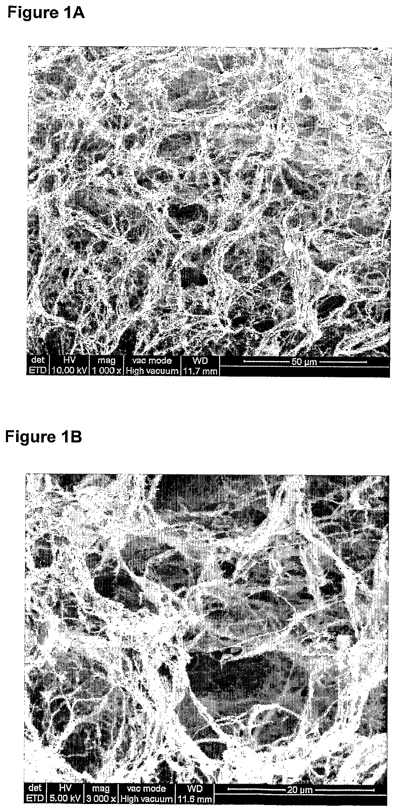

- La Figure 1 représente des images de microscopie électronique à balayage (microscope à effet de champ) d'un échantillon d'aérogel obtenu selon le procédé de l'invention aux grossissements x 1000 (Figure 1A), x 3000 (Figure 1B) et x 60000 (Figure 1C).
- La Figure 2 représente une photographie d'un aérogel obtenu selon le procédé de l'invention.
- La Figure 3 représente des spectres Raman de nanotubes de carbone bruts et d'un aérogel de la présente invention.
- La Figure 4 représente la distribution du diamètre des nanotubes de carbone observée après séchage de la solution de nanotubes de carbone individualisés réduits obtenue à l'issue de l'étape (b) du procédé de l'invention (courbe étroite de gauche) par rapport aux nanotubes de carbone brut en faisceaux (courbe large de droite).

### EXEMPLES

Sauf indication contraire, toutes les expérimentations sont effectuées sous atmosphère inerte, par exemple sous argon ou azote. En particulier, les manipulations sont effectuées en boîte à gants sous atmosphère d'argon sec (teneur en O₂ < 10 ppm, teneur en H₂O < 10 ppm)

### Exemple 1 : Préparation d'un aérogel de nanotubes de carbone à partir d'une solution d'un sel polyélectrolyte de nanotubes de carbone dans DMSO

La préparation d'une solution de sel polyélectrolyte de nanotubes de carbone peut être effectuée selon la méthode décrite dans WO 2005/073127 [ref 35]. Une variante de cette méthode est décrite ci-dessous :

### Préparation d'un sel de potassium de naphtalène (Naph⁻ K⁺)

100 mg de naphtalène sont placés dans un ballon de 250 cm³ auquel on rajoute 30 mg de potassium en petits morceaux de surface brillante (épluchés au scalpel juste avant utilisation), puis de l'ordre de 100 cm3 de THF. Le ballon est chauffé au reflux jusqu'à ce que la solution prenne une couleur vert très sombre et est laissé au reflux quelques heures.

### Réduction de nanotubes de carbone

La solution obtenue ci-dessus est ensuite versée en filtrant, pour éviter un excès de potassium solide, sur 55 mg de nanotubes bruts (synthétisés à l'arc électrique). Le tout est laissé sous agitation magnétique à température ambiante pendant une quinzaine d'heures. Alternativement, on peut suivre la diminution de la concentration en Naph-K⁺ par spectroscopie UV visible. Le mélange réactionnel est filtré sur membrane de type Millipore® (0,45 microns de porosité). Le solide est rincé plusieurs fois avec du THF (distillé sur un mélange potassium/naphtalène), jusqu'à ce que le THF reste incolore après passage à travers le filtre. Le solide est ensuite séché sous vide à température ambiante. Le solide présente une bonne stabilité au stockage d'au moins plusieurs mois, sous atmosphère contrôlée.

### Préparation d'une solution de nanotubes de carbone individualisés réduits dans du DMSO

40 mg de sel de nanotubes obtenu ci-dessus sont soumis à agitation magnétique pendant environ 15 heures dans 16 cm³ de DMSO à température ambiante. La solution obtenue est centrifugée à 4000 t/min pendant 1 heure, puis décantée. Une solution homogène de nanotubes de carbones individualisés, c'est-à-dire ne comprenant pas d'agrégats visibles au microscope optique (grossissement = 400). Ladite solution contient 2 mg de nanotubes de carbone réduits par gramme de DMSO.

### Lyophilisation de la solution de nanotubes de carbone individualisés réduits dans du DMSO

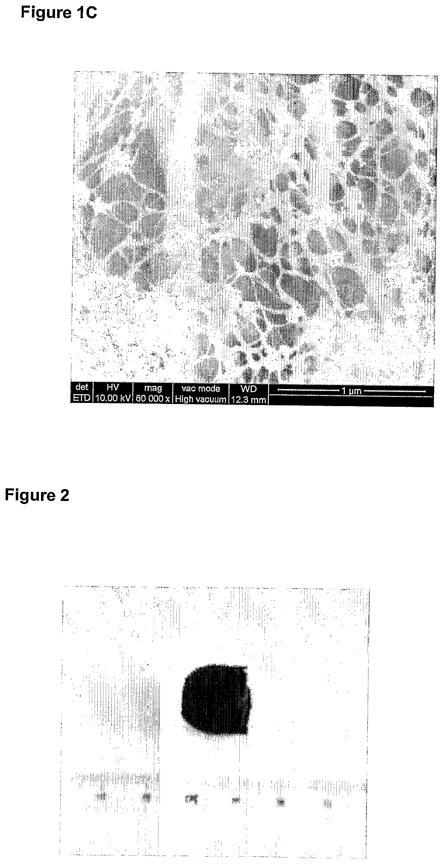

De 1 à 6 g de la solution précédente sont placés dans un tube à essai en verre, muni d'une vanne qui (i) permet d'isoler hermétiquement le contenu du tube à essai, (ii) possède un mode de connexion à la pompe à vide (de type swageloke par exemple). Le tube est rempli de solution sous atmosphère inerte, la vanne est fermée et le tube peut alors être sorti de l'enceinte à atmosphère inerte. Le tube est optionnellement plongé dans l'azote liquide pour congeler brutalement la solution. Le tube est ensuite placé dans un bain thermostaté à une température inférieure à celle de congélation du solvant (par exemple à 6°C avec du DMSO) et l'autre côté de la vanne est connecté à la pompe à vide. Le contenu du tube à essai est alors mis sous vide par ouverture de la vanne (10⁻⁵ mbars). Après une quinzaine d'heures (pour 1 g de solution) ou une soixantaine d'heures (4 à 6 g de solution), [typiquement une nuit ou un week-end], on observe un solide vaporeux et sec en lieu et place de la solution (voir figure 2). Celui-ci peut être alors récupéré par ouverture du tube à essai. Alternativement, si l'on veut garder l'aérogel sous atmosphère inerte, suivant ce que l'on veut en faire après, on ferme la vanne, on remet le tube avec la vanne fermée dans la boîte à gants à atmosphère contrôlée et l'on ouvre le tube sous atmosphère inerte.

Les protocoles de l'Exemple 1 ci-dessus, effectués à partir de nanotubes de carbone monoparois synthétisés à l'arc électrique, ont également été effectués avec des nanotubes de carbone monoparois fabriqués à partir du procédé HiPCO (procédé monoxyde de carbone haute-pression « High Pressure CO »), des nanotubes de Swan (multiparois de 2 à 4 parois), et des multiparois de la société Arkema, avec des résultats similaires.

### Exemple 2 : Mesure de la densité apparente de l'aérogel de l'Exemple 1

### Mesure du volume de l'aérogel :

L'aérogel est préparé selon l'Exemple 1 à partir d'un sel de sodium de nanotubes monoparois synthétisés à l'arc électrique. 4 ml de solution de nanotubes de carbone individualisés réduits dans du DMSO sont lyophilisés dans un tube à essai. Une marque est faite sur la paroi du tube à essai au niveau de la surface supérieure de l'aérogel.

Après avoir retiré l'aérogel du tube à essai, un liquide y est introduit jusqu'au niveau de la marque précitée. Le volume du liquide est ensuite mesuré dans une éprouvette graduée : 2,5 ml. Alternativement, le liquide peut être pesé, et son volume déterminé à partir de sa densité.

### Mesure de la masse de l'aérogel

L'aérogel retiré du tube à essai ci-dessus est pesé : 6,0 mg.

### Densité apparente

La densité apparente de l'aérogel est calculée par le rapport masse/volume déterminés ci-dessus : 6,0/2,5 = 2,4 mg/cm³.

### Exemple 3 : Mesure du volume occupé par les pores de l'aérogel de l'Exemple 2

La densité des nanotubes monoparois étant de 1.4 g/cm³, une masse de 6.0 mg conduit à un volume V = m/d = 6.0 10⁻³/ 1.4 = 4.3 10⁻³ cm³.

Le pourcentage du volume des pores se déduit alors simplement comme suit : (Vglobal - VNT)/Vglobal, Vglobal représentant le volume de l'aérogel tel que déterminé à l'Exemple 2.

Ainsi, pour l'aérogel de l'Exemple 2, le % du volume des pores est : (2.5 - 0.0043)/2.5 = 99.8 %.

### Exemple 4 : Mesure de la surface spécifique de l'aérogel de l'Exemple 2

La mesure de surface spécifique a été faite par adsorption de Krypton et par la méthode BET.

La surface spécifique déterminée par adsorption de Krypton: 311 m²/g.

La surface spécifique déterminée par la méthode BET est : 345 m²/g.

Il faut comprendre que les deux méthodes donnent une valeur identique.

### Exemple 5 : Mesure de la résistivité électrique de l'aérogel de l'Exemple 1

Afin de s'affranchir des problèmes de résistances de contact, les mesures de résistivité électrique des aérogels ont été effectuées par la méthode dite "en quatre points".

Des aérogels de nanotubes de carbone ont été préparés selon l'Exemple 1 à partir de trois sources de nanotubes différentes : des nanotubes dits "Elicarb" commercialisés par la socité Thomas Swan, des nanotubes synthétisés par la méthode de l'arc électrique et des nanotubes dits "HipCO".

Autour d'un échantillon de forme cylindrique, quatre anneaux ont été formés à la laque d'argent, afin d'assurer une bonne géométrie des lignes de champ. Les mesures de résistance ont montré une conductivité de 5.0 S/cm pour un aérogel de nanotubes dits "Elicarb", 0.3 S/cm pour des nanotubes synthétisés par la méthode de l'arc électrique et 0.03 S/cm pour des nanotubes dits "HipCO".

### Exemple 6 : Préparation d'un aérogel de nanotubes de carbone à partir d'une solution d'un sel polyélectrolyte de nanotubes de carbone et de PMMA dans le DMSO

Une solution de nanotubes de carbone individualisés réduits dans du DMSO est préparée selon le protocole décrit dans l'Exemple 1 à partir de nanotubes de carbone dits "Elicarb" commercialisés par la société Thomas Swan. 6% en poids (par rapport aux nanotubes de carbone) de polyméthyl méthacrylate (PMMA) est ajouté à la solution. Le mélange résultant est ensuite lyophilisé selon le protocole décrit dans l'Exemple 1.

### Exemple 7 : Mesure de la résistivité électrique de l'aérogel de l'Exemple 6

La résistivité de l'aérogel de l'Exemple 6 a été déterminée selon le protocole de l'Exemple 5.

L'aérogel obtenu à partir d'une solution comprenant du PMMA et du sel de nanotubes de carbone a une conductivité inférieure à celle observée pour les aérogels de l'Exemple 5. En l'occurrence, un aérogel contenant 6% en poids de PMMA a une conductivité de 0.05 S/cm au lieu de 5 S/cm.

### Exemple 8 : Mise en évidence de la biocompatibilité d'un aérogel de l'invention

Le principe expérimental consiste à comparer le comportement de cellules mises en culture au contact d'un aérogel à des cellules 'témoin' : sans aérogel (substrat favorable à la croissance cellulaire, par exemple, des plaques de culture de laboratoire).

Au temps T0, la même quantité de cellules est mise en culture, au contact ou non de l'aérogel (un échantillon *test* et un échantillon *témoin* respectivement) ; aux temps T1, T2... (7 jours, 14jours...par exemple) la prolifération cellulaire est alors mesurée pour chaque échantillon.

On considérera que le matériau est biocompatible et/ou qu'il y a une absence de cytotoxicité lorsque les valeurs obtenues pour l'échantillon test seront supérieures ou égales à 75% des valeurs de l'échantillon témoin.

Pour quantifier la prolifération cellulaire, le test MTS (Promega's CellTiter 96 AQueous One Solution Cell Proliferation Assay, promega) peut être utilisé.Le principe de ce test est de mesurer la bioréduction du sel de tétrazolium en un produit coloré-le formazan, par les cellules vivantes.

La quantification peut se faire directement en comptant les cellules ou indirectement par changement colorimétrique traduisant la modification du substrat (mesures de densité optique).

### Listes des références

[1] *"*Les aérogels et le structure alvéolaires : deux exemples de mousses de carbone" de L. Kocon et T. Piquero, dans L'actualité Chimique, N° 245-246, pp. 119-123 (mars-avril 2006)
[2] ["Nouveaux concepts d'élaboration de matériaux carbonés poreux" de C. Vix-Guterl, J. Parmentier, P. Delhaés, dans L'actualité chimique, n° 245-246, pp 124-128 (mars-avril 2006)
[3] *"*Synthesis of highly ordered carbon molecular sieves via template-mediated structural transformation" de R. Ryoo, S.-H. Soo, S. Jun, dans The Journal of Physical chemistry B, 103 (37), pp. 7743-7746 (1999)
[4] ["High-thermal conductivity, mesophase pitch-derived carbon foams : effect of precursors on structure and properties" de J. Klett et col., dans Carbon, 38, pp. 153-173 (2000)
[5] *"*Novel high strength graphitic foams", de T. Beechem, K. Lafdi, dans Carbon, 44, pp. 1548-1549 (2002)
[6] *["*Fabrication of nano-structure control of carbon aerogels via microemulsion templatted sol-gel polymerisation method", de D. Wu, R. Fu, M.S. Dresselhaus, G. Dresselhaus, dans Carbon, 44, pp 675-680 (2005)
[7] *"*Preparation and properties of resorcinol formaldehyde organic and carbon gels", de S.A. Al-Muthtsabeb, J.A. Ritter, dans Adv. Mater., 15(2), pp. 101-104 (2003)
[8] Schaffer, M. S. P., Windle, A. H., "Fabrication and Characterization of Carbon Nanotube/poly (vinyl alcohol) Composites", Adv. Mater., 11, pp 937-941 (1999)
[9] "Nanotubes from carbon" de P.M. Ajayan (Chem. Rev., vol. 99, p.1787, 1999)
[10] Aldissi, M.; Schmitz, B.; Lazaro, E.; Bhamidipati, M.; Dixon, B., "Conducting Polymers In Ultracapacitor Applications", 56.sup.th Annu. Tech. Conf.-Soc. Plast. Eng., (Vol. 2), pp 1197-1201 (1998)
[11] An, K. H.; Kim, W. S.; Park, Y. S.; Moon, J.-M.; Bae, D. J.; Lim, S. C.; Lee, Y. S.; Lee, Y. H. "Electrochemical Properties Of High-Power Supercapacitors Using Single-Walled Carbon Nanotube Electrodes", Adv. Funct. Mater. 11, pp 387-392 (2001)
[12] Yu, R., Chen, L., Liu, Q., Lin, J., Tan, K. -L., Ng, S. C., Chan, H. S. O., Xu, G.-Q.,Hor, T. S. A. "Platinum Déposition On Carbon Nanotubes Via Chemical Modification", Chem. Mater. 10, pp 718-722 (1998)
[13] Planeix, J. M.; Coustel, N.; Coq, B.; Brotons, V.; Kumbhar, P. S.; Dutartre, R.; Geneste, P.; Bernier, P.; Ajayan, P. M., "Application Of Carbon Nanotubes As Supports_in Heterogeneous Catalysis", J. Am. Chem. Soc. 116, pp 7935-7936 (1994)
[14] Tans, S. J., Verschueren, A. R. M., Dekker, C., "Room-Temperature Transistor Based On A Single Carbon Nanotube", Nature 393, pp 49-52 (1998)
[15] Bachtold, A.; Hadley, P.; Nakanishi, T.; Dekker, C., "Logic Circuits With Carbon Nanotube Transistors". Science 294 pp 1317-1320 (2001)
[16] Yodh et al., « Carbon nanotube aerogels », Advanced Materials, 2007, 19, pp. 661-664
[17] Backov et al., Demande de brevet français N° 06/11143 (publication N° FR 2 910 458)
[18] « Synthesis of graphite intercalation compounds », A. Hérold in Chemical physics of intercalation, A.P. Legrand et S. Flandrois Eds, NATO ASI Series, series B, Vol. 172, pp. 3-45 (1987)
[19] C. Stein, J. Poulenard, L. Bonnetain, J. Golé, C.R. Acad. Sci. Paris 260, 4503 (1965)
[20] « Synthesis of graphite intercalation compounds », A. Hérold in Chemical physics of intercalation, A.P. Legrand et S. Flandrois Eds, NATO ASI Series, series B, Vol. 172, pp. 3-45 (1987)
[21] F. Béguin et R. Setton New ternary lamellar compounds of graphite, Carbon 13, 293-)295 (1975)
[22] Demande de brevet français N° FR 2 881 362
[23] Matyjaszewski, K. ; Eds. ; Advances in Controlled/Living Radical Polymerization, (American Chemical Society 2003)
[24] Hsieh, H. L. ; Quirk, R. P. ; Eds. ; Anionic Polymerization Principles and Practical Applications, (Marcel Dekker 1996)
[25] Matyjaszewski, K.; Davies, T. P; Eds.; Handbook of Radical Polymerization, (Wiley-Interscience 2002)
[26] Fontaine, L. ; Initiation à la Chimie et à la Physico-Chimie Macromoléculaires (Groupe Français d'Etudes et d'Applications des Polymères volume 12 (Chapitre 3))
[27] Chakraborty et al., « Functionalization of potassium graphite », Angew. Chem. Int. Ed., 46, 4486-4488 (2007)
[28] Stankovitch et al., « Graphene based composite materials », Nature, 442, 282-286 (2006)
[29] FR 04/05120
[30] WO 2006/136715
[31] Matyjaszewski, K. ; Eds. ; Cationic Polymerizations Mechanisms, Synthesis, and Applications, (Marcel Dekker 1996)
[32] FR 2 873 381
[33] "Chemistry of Carbon Nanotubes" de Dimitrios Tasis, Nikos Tagmatarchis, Alberto Bianco et Maurizio Prato, Chem. Rev. 2006, 106, 1105-1136
[34] Pénicaud et al., « Spontaneous dissolution of a single-wall carbon nanotube salt », J. Am. Chem. Soc., 127, 8-9, (2005)
[35] WO 2005/073127
[36] WO 2004/076381
[37] Lago et al., « Mechanical damage of carbon nanotubes by ultrasound », Carbon, 34(6) 814-816, (1996)
[38] Badaire et al., « In situ measurement of nanotube dimensions in suspensions by depolarized dynamic light scattering », Langmuir, 20 : 10367-10370 (2004)
[39] Heller et al., « Concomittant length and diameter separaton of single-walled carbon nanotubes », J. Am. Chem. Soc., 126 :14567-14573 (2004)
[40] Hennrich et al., « The mechanism of cavitation-induced scission of single-walled carbon nanotubes », J. Phys. Chem. B, 111 : 1932-1937 (2007)
[41] Islam et al., « High weight fraction surfactant solubilization of single-wall carbon nanotubes in water », Nanoletters, Vol. 3(2) : 269-273 (2003)

## Revendications

1. Procédé de préparation d'un aérogel de nanotubes de carbone individualisés comprenant les étapes suivantes réalisées sous atmosphère inerte :
(a) réduction de nanotubes de carbone par un métal alcalin pour conduire à un sel polyélectrolyte de nanotubes de carbone ;
(b) exposition dudit sel polyélectrolyte de nanotubes de carbone à un solvant polaire aprotique pour conduire à une solution de nanotubes de carbone individualisés réduits ;
(c) congélation de ladite solution de nanotubes individualisés ; et
(d) sublimation du solvant ;
le procédé étant réalisé sans altération structurale des nanotubes de carbone par sonication et en l'absence de tensioactif.

2. Procédé selon la revendication 1, dans lequel l'étape de réduction a) comprend l'addition aux nanotubes de carbone, sous atmosphère inerte, d'un sel polyaryl alcalin de formule A⁺B⁻, dans laquelle :
A⁺ représente un cation d'un ion alcalin, et
B⁻ représente un anion d'un composé polyaromatique.

3. Procédé selon la revendication 2, dans lequel le composé polyaromatique est choisi dans le groupe comprenant le naphtalène, le phénanthrène, le biphényle, l'anthracène, le pérylène, la benzophénone, la fluorénone, la benzoquinone et l'anthraquinone.

4. Procédé selon l'une quelconque des revendications 1 à 3, dans lequel le solvant polaire aprotique est le sulfolane, le diméthylsulfoxyde, le diméthylformamide, la N-méthylpyrrolidone ou le N-méthylformamide.

5. Aérogel de nanotubes de carbone individualisés obtenu par un procédé selon l'une quelconque des revendications 1 à 4.

6. Utilisation d'un aérogel selon la revendication 5, pour la préparation de matériaux composites.

7. Utilisation selon la revendication 6, dans laquelle le matériau composite est obtenu par un procédé comprenant une étape e) consistant à imbiber ledit aérogel avec un polymère, un mélange de polymères ou une résine, sous forme liquide ou en solution, ou du silicium en fusion.

8. Utilisation selon la revendication 6, dans laquelle le matériau composite est obtenu par un procédé comprenant une étape b2) de mélange d'une solution de polymère, d'un mélange de polymères, ou d'une résine avec la solution de nanotube de carbone individualisés réduits obtenue à l'étape b).

9. Utilisation selon la revendication 8, dans laquelle le polymère est le polymethyl methacrylate ou « PMMA ».

10. Utilisation d'un aérogel selon la revendication 5, pour la préparation de composants électrochimiques.

11. Utilisation selon la revendication 10, dans laquelle le composant électrochimique est un supercondensateur, un biocapteur ou une électrode de pile à combustible.

12. Utilisation d'un aérogel selon la revendication 5, pour la préparation de matériaux de filtration et/ou dépollution.

13. Utilisation d'un aérogel de carbone selon la revendication 12, dans laquelle le matériau de dépollution est une membrane pour le traitement des eaux ou des marées noires.

14. Utilisation d'un aérogel selon la revendication 5, pour la préparation de biomatériaux.

15. Utilisation d'un aérogel de carbone selon la revendication 14, dans laquelle le biomatériau est un support pour la croissance cellulaire, pour la croissance osseuse ou pour le remplacement de cartilage.

16. Utilisation d'un aérogel selon la revendication 5, pour la préparation de supports de catalyseur pour la catalyse hétérogène.

## Patentansprüche

1. Verfahren zur Herstellung eines Aerogels aus individualisierten Kohlenstoffnanoröhren, das die folgenden unter inerter Atmosphäre durchgeführten Schritte umfasst:
(a) Reduktion von Kohlenstoffnanoröhren durch ein Alkalimetall, um ein Kohlenstoffnanoröhren-Polyelektrolytsalz zu erhalten;
(b) Aussetzen des Kohlenstoffnanoröhren-Polyelektrolytsalzes gegenüber einem polaren aprotischen Lösungsmittel, um eine Lösung von reduzierten individualisierten Kohlenstoffnanoröhren zu erhalten;
(c) Gefrieren der Lösung von individualisierten Nanoröhren und
(d) Sublimation des Lösungsmittels,
wobei das Verfahren ohne Strukturveränderung der Kohlenstoffnanoröhren durch Ultraschallbehandlung und in Abwesenheit von Tensid durchgeführt wird.

2. Verfahren nach Anspruch 1, wobei der Reduktionsschritt a) die Zugabe eines Alkalimetallpolyaryl-Salzes der Formel A⁺B⁻ unter inerter Atmosphäre zu den Kohlenstoffnanoröhren umfasst, wobei:
A⁺ ein Kation eines Alkalimetallions darstellt und
B⁻ ein Anion einer polyaromatischen Verbindung darstellt.

3. Verfahren nach Anspruch 2, wobei die polyaromatische Verbindung aus der Gruppe ausgewählt ist, die Naphthalin, Phenanthren, Biphenyl, Anthracen, Perylen, Benzophenon, Fluorenon, Benzochinon und Anthrachinon umfasst.

4. Verfahren nach einem der Ansprüche 1 bis 3, wobei das polare aprotische Lösungsmittel Sulfolan, Dimethylsulfoxid, Dimethylformamid, N-Methylpyrrolidon oder N-Methylformamid ist.

5. Aerogel aus individualisierten Kohlenstoffnanoröhren, das durch ein Verfahren nach einem der Ansprüche 1 bis 4 erhalten wird.

6. Verwendung eines Aerogels nach Anspruch 5 zur Herstellung von Verbundwerkstoffen.

7. Verwendung nach Anspruch 6, wobei der Verbundwerkstoff durch ein Verfahren erhalten wird, das einen Schritt e) umfasst, in dem man das Aerogel mit einem Polymer, einem Gemisch von Polymeren oder einem Harz in flüssiger Form oder in Lösung oder mit schmelzflüssigem Silizium imprägniert.

8. Verwendung nach Anspruch 6, wobei der Verbundwerkstoff durch ein Verfahren erhalten wird, das einen Schritt b2) des Mischens einer Lösung eines Polymers, eines Gemischs von Polymeren oder eines Harzes mit der in Schritt b) erhaltenen Lösung von reduzierten individualisierten Kohlenstoffnanoröhren umfasst.

9. Verwendung nach Anspruch 8, wobei das Polymer Polymethylmethacrylat oder "PMMA" ist.

10. Verwendung eines Aerogels nach Anspruch 5 zur Herstellung elektrochemischer Komponenten.

11. Verwendung nach Anspruch 10, wobei die elektrochemische Komponente ein Superkondensator, ein Biosensor oder eine Brennstoffzellenelektrode ist.

12. Verwendung eines Aerogels nach Anspruch 5 zur Herstellung von Filtrations- und/oder Säuberungsmaterialien.

13. Verwendung eines Kohlenstoff-Aerogels nach Anspruch 12, wobei das Säuberungsmaterial eine Membran zur Behandlung von Wasser oder Ölverschmutzungen ist.

14. Verwendung eines Aerogels nach Anspruch 5 zur Herstellung von Biowerkstoffen.

15. Verwendung eines Kohlenstoff-Aerogels nach Anspruch 14, wobei der Biowerkstoff ein Träger für das Zellwachstum, für das Knochenwachstum oder für Knorpelersatz ist.

16. Verwendung eines Aerogels nach Anspruch 5 zur Herstellung von Katalysatorträgern für die heterogene Katalyse.

## Claims

1. A process for preparing an aerogel of individualized carbon nanotubes comprising the following steps carried out under an inert atmosphere:
(a) reducing carbon nanotubes with an alkali metal to lead to a polyelectrolyte salt of carbon nanotubes;
(b) exposing said polyelectrolyte salt of carbon nanotubes to a polar aprotic solvent to lead to a solution of individualized reduced carbon nanotubes;
(c) freezing said solution of individualized nanotubes; and
(d) sublimating the solvent;
the process being carried out without structural alteration of the carbon nanotubes by sonication and in the absence of surfactant.

2. The process according to claim 1, wherein the reduction step a) comprises the addition, to the carbon nanotubes, under an inert atmosphere, of an alkali metal polyaryl salt of formula A⁺B⁻, wherein:
A⁺ represents a cation of an alkali metal ion, and
B⁻ represents an anion of a polyaromatic compound.

3. The process according to claim 2, wherein the polyaromatic compound is selected from the group comprising naphthalene, phenanthrene, biphenyl, anthracene, perylene, benzophenone, fluorenone, benzoquinone and anthraquinone.

4. The process according to any one of claims 1 to 3, wherein the polar aprotic solvent is sulfolane, dimethyl sulfoxide, dimethylformamide, N-methylpyrrolidone or N-methylformamide.

5. An aerogel of individualized carbon nanotubes obtained by a process according to any one of claims 1 to 4.

6. Use of an aerogel according to claim 5, for the preparation of composites.

7. The use according to claim 6, wherein the composite is obtained by a process comprising a step e) of impregnating said aerogel with a polymer, a blend of polymers or a resin, in liquid form or in solution, or molten silicon.

8. The use according to claim 6, wherein the composite is obtained by a process comprising a step b2) of mixing a polymer solution, a blend of polymers, or a resin with the solution of individualized reduced carbon nanotubes obtained in step b).

9. The use according to claim 8, wherein the polymer is polymethyl methacrylate or "PMMA".

10. Use of an aerogel according to claim 5, for the preparation of electrochemical components.

11. The use according to claim 10, wherein the electrochemical component is an ultracapacitor, a biosensor or a fuel cell electrode.

12. Use of an aerogel according to claim 5, for the preparation of filtration and/or pollution-control materials.

13. The use of a carbon aerogel according to claim 12, wherein the pollution-control material is a membrane for water treatment or for the treatment of oil spills.

14. Use of an aerogel according to claim 5, for the preparation of biomaterials.

15. The use of a carbon aerogel according to claim 14, wherein the biomaterial is a support for cell growth, for bone growth or for cartilage replacement.

16. Use of an aerogel according to claim 5, for the preparation of catalyst supports for heterogeneous catalysis.
